# FASCICULE DE BREVET EUROPEEN

(11) **EP 0 390 684 B1**
(45) Date de publication et mention de la délivrance du brevet: **24.02.1993**
(21) Numéro de dépôt: 90400849.7
(22) Date de dépôt: 29.03.1990
(51) Int. Cl.: A61M 16/00

(54) **Dispositif d'assistance respiratoire**
Vorrichtung zur Unterstützung der Atmung
Respiratory assistance device

(30) Priorité: 31.03.1989 FR 8904280
(43) Date de publication de la demande: 03.10.1990
(73) Titulaire: Boussignac, Georges, F-92160 Antony (FR); Labrune, Jean-Claude, F-92160 Antony (FR)
(72) Inventeur: Boussignac, Georges, F-92160 Antony (FR); Labrune, Jean-Claude, F-92160 Antony (FR)
(74) Mandataire: Bonnetat, Christian

(56) Documents cités:
- EP-A- 0 153 991
- EP-A- 0 245 142
- DE-C- 160 709
- FR-A- 2 613 639
- GB-A- 2 057 273

## Description

La présente invention a pour objet un dispositif pour assistance respiratoire, conformément à la préambule de la revendication 1, utilisable sur des patients dont la respiration spontanée est absente ou insuffisante, qu'ils soient placés ou non sous respiration artificielle. Un tel dispositif est connu du document EP-A-0 245 142.

On connaît divers dispositifs, tels que des masques, des sondes ou des canules orales, nasales, endotrachéales, trachéotomiques, destinés à faire la jonction entre un appareil de respiration artificielle et/ou d'anesthésie et le système respiratoire d'un patient. Ces dispositifs, essentiellement en forme de tubes, peuvent, selon le cas, comporter des moyens d'immobilisation tels que des pattes ou des collerettes au voisinage de l'extrémité proximale, pour le maintien sur la bouche ou le nez du patient, ou encore des ballonnets gonflables au voisinage de l'extrémité distale, pour le maintien par friction dans la trachée.

Les dispositifs connus présentent des inconvénients importants. Ainsi, par exemple, lorsqu'un tube de type connu est déconnecté du respirateur artificiel et que le patient a besoin d'air enrichi en oxygène, il est nécessaire d'introduire dans ledit tube une sonde reliée à une source d'oxygène. Par ailleurs, dans les cas de respiration spontanée insuffisante, le patient doit nécessairement rester relié au respirateur jusqu'au rétablissement complet de sa respiration spontanée.

Aussi, pour remédier à ces inconvénients, on a déjà proposé (EP-A-0245142) des dispositifs d'assistance respiratoire qui, outre le canal principal formé par le tube, comportent au moins un canal auxiliaire, par exemple ménagé dans la paroi dudit tube, permettant l'injection d'un jet de gaz respirable (oxygène, air ou mélange air-oxygène) destiné à la ventilation du patient, ce canal auxiliaire débouchant dans le canal principal au voisinage de l'extrémité distale de ce dernier.

On connaît aussi (GB-A- 2057 273) un dispositif d'assistance respiratoire, qui comporte un canal auxiliaire en forme d'un tube incliné vers l'intérieur du canal principale.

Toutefois, ces dispositifs d'assistance respiratoire à canal auxiliaire d'injection de gaz respirable présentent l'inconvénient majeur que ledit jet de gaz respirable vient frapper directement la muqueuse, de sorte que celle-si est traumatisée.

La présente invention a pour objet principal de remédier à cet inconvénient.

A cette fin, l'invention propose un dispositif d'assistance respiratoire conformément à la revendication 1.

Ainsi, le jet de gaz respirable sous pression traversant ledit canal auxiliaire est défléchi vers l'axe du canal principal, lorsqu'il pénètre dans celui-ci. En aval desdits moyens de déflexion, c'est-à-dire à l'intérieur du canal principal, la pression dudit jet de gaz respirable chute et le jet sort à faible pression à travers l'orifice distal du tube. L'expérience a montré qu'en aval de la sortie distale du tube, la pression est faible et maintenue constante dans tout l'espace respiratoire. Cette pression est dépendante du débit de gaz respirable dans les canaux auxiliaires.

Par suite, avec le dispositif d'assistance respiratoire conforme à l'invention, on peut par exemple apporter de l'oxygène et un mélange air-oxygène directement dans les poumons, à hauteur de la carène, et supprimer ainsi l'espace mort qui existe dans les sondes actuelles et qui est d'environ un tiers du volume respiratoire total pour un adulte et d'environ la moitié pour les nouveaux nés prématurés.

La suppression de cet espace mort correspond à une augmentation de performance du cycle respiratoire de plus de 25% dans tous les cas de malades et de près de 50% dans certains cas.

De plus, lorsque plusieurs canaux auxiliaires sont ménagés dans la paroi dudit tube, il est préférable que les orifices distaux desdits canaux auxiliaires soient formés dans une première face annulaire évasant ledit canal principal et que lesdits moyens de déflexion soient formés par une seconde face annulaire, disposée en regard de ladite première face et convergente en direction de l'orifice distal dudit canal principal.

En aval de ladite seconde face, entre celle-ci et l'orifice distal du canal principal, ce dernier peut être cylindrique. Toutefois, l'expérience a montré qu'il était préférable que la portion terminale du canal principal soit légèrement évasée. Aussi, selon une autre particularité de l'invention, ladite seconde face est prolongée vers ledit orifice du canal principal par une paroi évasant légèrement ledit canal principal.

Les moyens de déflexion peuvent être formés directement dans la paroi interne dudit tube. Toutefois, à des fins de facilité de fabrication, il est avantageux que lesdits moyens de déflexion soient formés sur un embout rapporté à l'extrémité distale dudit tube.

Lorsque le dispositif selon l'invention comporte une pluralité de canaux auxiliaires, il est avantageux qu'au moins certains d'entre eux soient alimentés en commun en gaz respirable. Une telle alimentation en commun desdits canaux peut se faire par l'intermédiaire d'une bague de distribution, coaxiale audit tube. Par ailleurs, lesdits canaux auxiliaires non alimentés en commun peuvent servir à l'introduction de produits gazeux additionnels, tels que des produits médicamenteux ou de l'humidité.

Pour pouvoir obtenir un dispositif à double flux permettant de favoriser l'expiration aussi bien que l'inspiration d'un patient, le dispositif selon l'invention peut comporter au moins un canal auxiliaire supplémentaire, indépendant dudit canal auxiliaire et relié à une source de gaz sous pression, ledit canal auxiliaire supplémentaire débouchant dans ledit canal principal au voisinage de l'extrémité proximale de ce dernier, et au moins l'extrémité proximale dudit canal auxiliaire supplémentaire débouchant dans le canal principal est parallèle à celui-ci, tandis que, en regard de l'orifice proximal dudit canal auxiliaire supplémentaire, sont prévus des moyens pour la déflexion du jet de gaz traversant ce dernier en direction de l'intérieur dudit canal principal.

De préférence, dans ce dernier mode de réalisation, la partie proximale du dispositif est semblable sinon identique, à la partie distale dudit dispositif, au moins en ce qui concerne la disposition desdits canaux auxiliaires et lesdits moyens de déflexion.

Ainsi, on voit que le dispositif conforme à l'invention permet :
. l'intubation sans arrêt de l'assistance respiratoire,
. l'injection de médicaments, d'anesthésiques ou d'humidité pendant l'assistance respiratoire,
. la mesure dynamique des pressions, car il suffit de prévoir des canaux auxiliaires dans lesquels sont disposées des sondes appropriées,
. l'augmentation du volume échangé, car la pression est autolimitée et il n'y a aucun risque d'écrasement des capillaires pulmonaires,
. la diminution pour une même quantité d'oxygène échangée de l'importance d'oxygène dans le mélange, ce qui diminue d'autant les effets secondaires de l'assistance,
. la possibilité d'utiliser des respirateurs moins coûteux que les respirateurs actuels.

Ce dispositif d'assistance respiratoire selon l'invention permet d'utiliser le différentiel de pression minimum nécessaire en apportant un volume de fluide respiratoire maximum.

Par ailleurs, la configuration physique du dispositif de l'invention permet de conserver un diamètre intérieur suffisant pour permettre le passage des instruments et des sondes médicales que l'opérateur veut utiliser.

Les figures du dessin annexé feront bien comprendre comment l'invention peut être réalisée. Sur ces figures des références identiques désignent des éléments semblables.

La figure 1 est une vue schématique et partielle, en coupe axiale agrandie, d'un premier mode de réalisation du dispositif de l'invention.

Les figures 2 et 3 sont des coupes tranversales, respectivement selon les lignes II-II et III-III de la figure 1.

Les figures 4 et 5 illustrent, schématiquement en coupe axiale agrandie, deux variantes de réalisation pour l'extrémité distale du dispositif selon l'invention.

La figure 6 montre, en coupe axiale schématique et partielle, une variante à double flux du dispositif selon la présente invention.

La figure 7 montre, en coupe axiale, une autre variante de réalisation du dispositif de l'invention, où le tube principal n'est plus destiné à être introduit directement dans une voie respiratoire, mais constitue l'embout d'un masque d'assistance respiratoire destiné à être appliqué sur le visage (bouche et/ou nez) d'un patient.

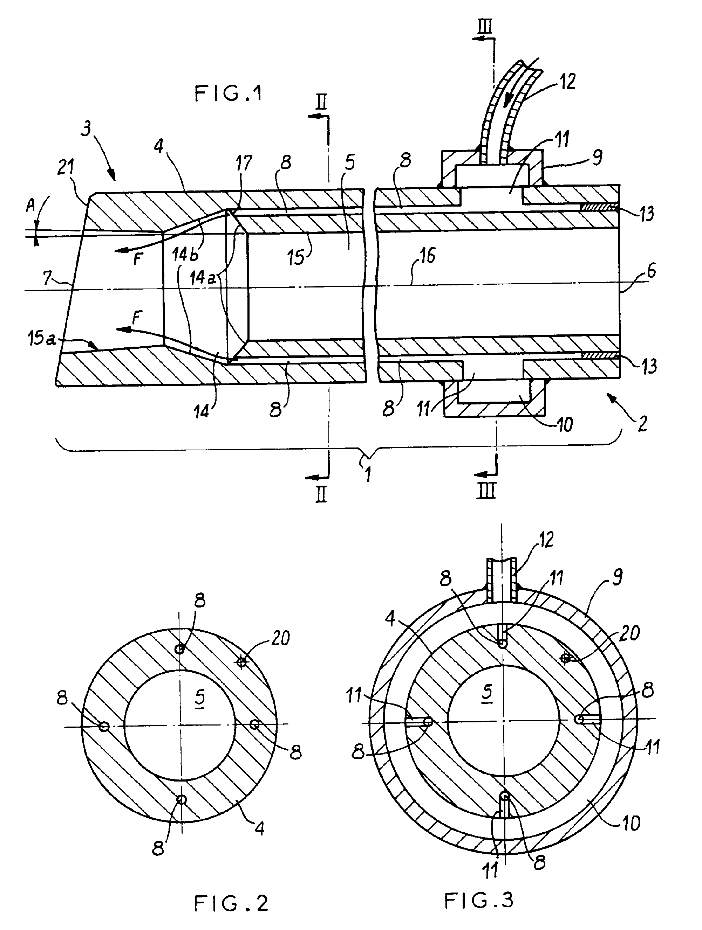

Sur la figure 1, on a représenté schématiquement et à grande échelle, les seules extrémités proximale 2 et distale 3 d'un mode de réalisation 1 du dispositif selon l'invention. Ce mode de réalisation peut constituer, par exemple, une sonde endotrachéale oro-nasale avec ou sans ballonnet, une sonde endotrachéale pédiatrique, une sonde de monitorage des gaz, une sonde endobronchique, une sonde nasopharyngée, une sonde d'intubation anatomique pour enfant, une sonde de Cole néonatale, une sonde canule de Guedel, ou une sonde nasale d'oxygénothérapie.

Le dispositif 1 comporte un tube 4, souple ou préformé (pour s'adapter à la morphologie du patient) délimitant un canal principal 5 débouchant, par l'orifice 6, à l'extrémité proximale 2, et, par l'orifice 7, à l'extrémité distale 3.

Ainsi, le canal principal 5 est capable d'assurer le passage entre les orifices 6 et 7, dont l'un (l'orifice 7) est destiné à se trouver à l'intérieur des voies respiratoires d'un patient et l'autre (l'orifice 6) est destiné à se trouver à l'extérieur dudit patient. Cet orifice 6 peut déboucher à l'air libre et, dans ce cas, le patient peut inspirer de l'air frais et expirer l'air vicié à travers le canal principal 5. On peut également relier l'orifice 6 à une source de gaz respirable sous pression (non représentée) et prévoir un système de valves unidirectionnelles, pour que le patient inspire le gaz respirable de ladite source à travers ledit canal principal 5 et expire le gaz vicié à l'air libre, également à travers ce canal principal.

Le diamètre du canal principal 5 est de l'ordre de quelques millimètres. Des essais satisfaisants ont été effectués avec des diamètres de 3 mm, 7 mm et 8 mm.

Par ailleurs, dans l'épaisseur de la paroi du tube 4 sont ménagés des canaux auxiliaires 8, s'étendant sur la presque totalité de la longueur du canal principal 5. Ces canaux auxiliaires 8 sont destinés à être reliés à une source de gaz respirable sous pression (non représentée). Par exemple, cette pression est de quelques bars (1,2 ou 4 bars) et elle est réglable.

Comme cela est représenté sur les figures 1 et 3, la liaison à la source de gaz respirable sous pression peut être réalisée au moyen d'une bague 9, entourant de façon étanche le tube 4, du côté de l'extrémité proximale 2, et délimitant une chambre annulaire étanche 10 autour dudit tube. Les canaux auxiliaires 8 sont mis en communication avec la chambre 10 grâce à des arrachements locaux 11 de la paroi du tube 4 et ladite chambre 10 est reliée à ladite source de gaz respirable par une liaison 12. Bien entendu, les extrémités proximales des canaux 8 sont obturées, par exemple par des bouchons 13.

Les canaux auxiliaires 8 ont un diamètre plus petit que celui du canal principal 5. Le diamètre des canaux auxiliaires 8 est de préférence inférieur à 1 mm et, de façon avantageuse, il est de l'ordre de 400 à 800 microns. Du côté distal, les canaux auxiliaires 8 débouchent dans un évidement 14 de la paroi interne 15 du tube 4. L'évidement 14 est annulaire et centré sur l'axe 16 de l'extrémité distale 3. Il comporte une face 14a, sensiblement transversale ou légèrement inclinée de façon à constituer un évasement du canal principal 5, dans laquelle débouchent lesdits canaux auxiliaires 8 par leurs orifices 17, ainsi qu'une face 14b suivant la face 14a et convergeant en direction de l'axe 16.

De préférence, entre la face inclinée convergente 14b et l'orifice distal 7, la paroi interne 15 présente une partie 15a légèrement évasée vers l'extérieur, comme cela est illustré par l'angle A sur la figure 1.

Ainsi, lorsque les canaux auxiliaires 8 sont alimentés en gaz respirable sous pression à travers les éléments 9 à 12, les jets gazeux correspondants heurtent la face inclinée 14b, qui les défléchit en direction de l'axe 16 (flèches F sur la figure 1) , engendrant au voisinage de celui-ci une zone de dépression favorisant la circulation gazeuse à l'intérieur du canal principal 5, de l'orifice proximal vers l'orifice distal. On favorise ainsi l'inspiration du patient.

De préférence, la distance entre chacun des orifices 17 et l'orifice 7 est de l'ordre de 1 à 2 cm.

En aval de l'orifice 7, la pression dans la cavité pulmonaire est faible et pratiquement constante.

Ainsi, grâce à l'invention, on obtient une assistance respiratoire non agressive pour le patient, avec disparition quasiment totale de l'espace mort inhérent aux sondes connues.

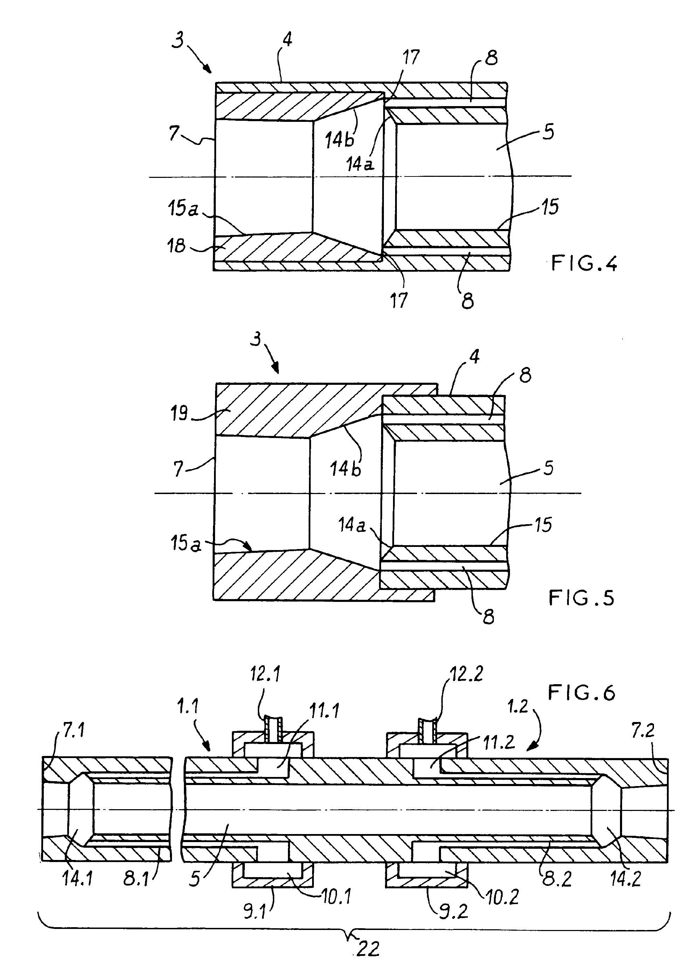

Dans le mode de réalisation de l'invention illustré par la figure 1, on a indiqué que l'ensemble des faces 14a et 14b était réalisé par évidement de la paroi interne 15 du canal principal 5. Il va de soi que ce mode opératoire n'est pas limitatif et que les faces 14a et 14b peuvent être obtenues de façons différentes. Par exemple, sur les figures 4 et 5, la face 14a est formée dans la paroi interne 15 du tube 4, alors que la face 14b est prévu sur un embout 18 ou 19 venant s'emboîter intérieurement (embout 18) ou extérieurement (embout 19) sur le tube 4.

Bien entendu, dans ce cas, l'orifice 7 et la paroi divergente 15a sont portés par l'embout 18 ou 19 correspondant.

Comme le montrent les figures 2 et 3, les canaux auxiliaires 8 sont disposés régulièrement autour de l'axe du tube 4. Leur nombre est variable suivant les utilisations (adulte ou enfant), mais il est généralement compris entre trois et neuf.

On remarquera que au moins l'un des canaux auxiliaires 8, au lieu d'être relié en commun avec les autres à la source de gaz respirable sous pression (par l'intermédiaire des éléments 9 à 12) peut être continuellement alimenté par la source de gaz respirable, de façon à maintenir une pression positive dans les poumons du patient et cela pendant ou à la fin de la phase expiratoire provoquée par l'insufflation de gaz dans les canaux auxiliaires 8 (effet anti-collapse).

Selon encore une autre variante de l'invention, un des canaux auxiliaires 8 peut également être spécialisé pour apporter un fluide médical ou un fluide d'humidification, si la source sous pression ne présente pas les caractéristiques requises.

Pour assurer l'humidification, le canal auxiliaire apportant de l'eau (tiède) est de préférence recourbé en forme de U à son extrémité distale, et vient déboucher dans une cavité ménagée dans la paroi interne 15, cavité dans laquelle débouche également un canal apportant de l'air sous pression. Dans ladite cavité, située de préférence entre l'évidement 14 et l'orifice distal 7, le canal d'eau et le canal d'air débouchent vis-à-vis l'un de l'autre, c'est-à-dire sensiblement sur le même axe, les deux fluides (air et eau) arrivant en sens contraires, ce qui permet la vaporisation de l'eau, la vapeur obtenue étant ensuite entraînée par l'air insufflé.

Au moins un canal supplémentaire 20 peut être prévu dans l'épaisseur du tube 4 afin de déboucher dans la face d'extrémité distale 21 du tube 4 et servir de logement à un dispositif de mesure de pression (non représenté).

Lorsque au moins deux prises de pression sont présentes, notamment à chacune des extrémités du tube, elles permettent, grâce à la différence des pressions mesurées, de calculer le débit gazeux.

Le tube 4 peut comporter à l'extrémité distale 3, un ballonnet gonflable (non représenté) muni des dispositifs nécessaires de sécurité ou tout autre ballonnet permettant de se comporter comme une soupape de sécurité en cas de surpression dans les poumons. Cet éventuel ballonnet peut être gonflé à partir d'un canal supplémentaire (non représenté) associé au tube 4.

Un dispositif de sécurité peut être constitué simplement d'un manchon élastique entourant le tube, partiellement collé à ce dernier, et recouvrant une perforation pratiquée à travers la paroi dudit tube, notamment au voisinage de l'extrémité proximale ; ainsi, lorsque la pression interne devient trop élevée, le gaz peut s'écouler à travers ladite perforation, puis entre la paroi externe du tube et la paroi interne du manchon élastique. Si un manchon de sécurité est également prévu au voisinage de l'extrémité distale, la perforation correspondante, étant placée au delà d'un éventuel ballonnet de maintien par friction, doit mettre en communication l'intérieur du tube avec l'air ambiant ; ledit ballonnet doit donc être contourné, ce que l'on obtient par exemple en le plaçant autour du manchon élastique.

La variante de réalisation 22 du dispositif selon l'invention, montrée par la figure 6, comporte deux dispositifs 1.1 et 1.2, chacun de structure semblable à celui (1) de la figure 1, accolés par leurs orifices 6, le dispositif 1.2 pouvant être plus court que le dispositif 1.1. Dans cette variante de réalisation 22, l'orifice distale (intérieur au patient) est constitué par l'orifice 7.1 du dispositif 1.1, tandis que l'orifice proximal (extérieur au patient) est formé par l'orifice 7.2 du dispositif 1.2. Chacun des dispositifs 1.1 et 1.2 est pourvu de son système d'alimentation 9.1 à 12.1 et 9.2 à 12.2 en gaz respirable sous pression, alimentant des canaux respectifs 8.1 ou 8.2, débouchant dans des évidements annulaires 14.1 ou 14.2, proches respectivement desdits orifices 7.1 et 7.2. Le dispositif 22 forme une sonde à double flux. Ce dispositif étant en place sur le patient (c'est-à-dire le dispositif 1.1 étant au moins partiellement introduit dans une voie respiratoire de celui-ci, alors que le dispositif 1.2 et les bagues 12.1 et 12.2 sont extérieures audit patient), on alimente alternativement les canaux 8.1 (à travers les éléments 9.1 à 12.1) et les canaux 8.2 (à travers les éléments 9.2 à 12.2) en gaz respirable sous pression, de manière à favoriser alternativement l'inspiration et l'expiration du patient.

A cet effet, un dispositif (non représenté) de commutation et de réglage des débits et des durées d'insufflation de gaz est relié aux bagues 9.1 et 9.2 d'une part, et à la source de gaz d'autre part.

Le tube respiratoire ainsi constitué permet une assistance respiratoire en insufflation et une assistance respiratoire en expiration, à condition de maintenir un débit continu assurant la sécurité et limitant le risque de collapsus pulmonaire.

La pression dans les canaux 8.2 est avantageusement plus élevée que la pression dans les canaux 8.1, car l'effet d'entraînement du fluide contenu dans la sonde n'est pas de même nature.

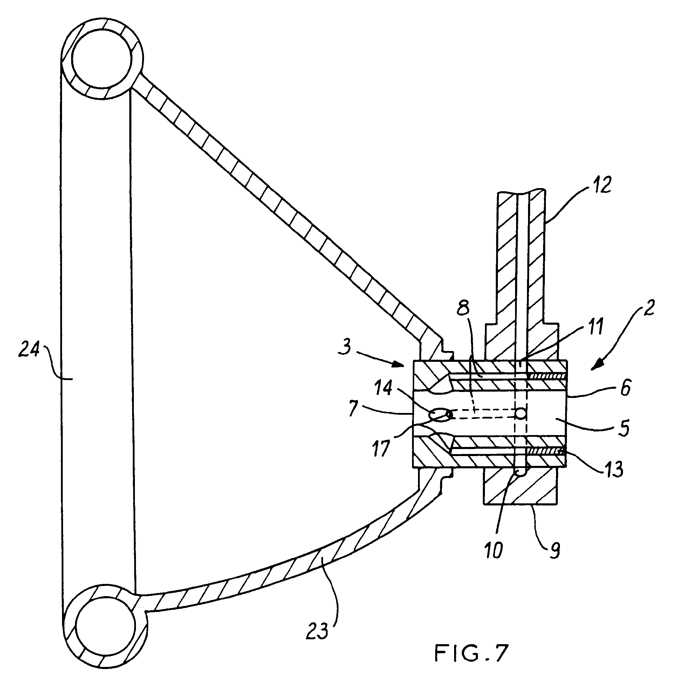

Le dispositif de la figure 7 se distingue des dispositifs des figures 1 à 6 d'une part par le fait que le tube 1 n'est pas destiné à être introduit directement dans une voie respiratoire, mais constitue l'embout d'un masque et, d'autre part, par le fait que les dimensions (longueur, diamètres) sont différentes. Selon la figure 7, le tube principal constitue l'embout d'entrée et de sortie d'air d'un masque qui, par ailleurs, est de type connu, c'est-à-dire comprenant essentiellement une coque 23, un bourrelet d'étanchéité 24, et des moyens (non représentés) de fixation tels que des sangles.

La figure 7 illustre aussi une variante de réalisation de l'évidement 14, les moyens de déflexion ne formant pas ici une gorge annulaire continue, mais étant constitués d'un ensemble discontinu d'évidements de forme générale conique, ménagés dans la paroi interne 15, et au fond de chacun desquels débouche l'extrémité distale d'un canal auxiliaire 8.

Le principe de fonctionnement du dispositif de la figure 7 est bien entendu celui décrit plus haut à propos des figures 1 à 6.

Par ailleurs, bien que la figure 7 comporte un tube où l'assistance respiratoire n'agit que dans le sens de l'inspiration (comme dans le cas du dispositif de la figure 1), un masque peut être également équipé d'un dispositif selon la figure 6, pour l'assistance à l'inspiration et à l'expiration.

Le tube 4 constitutif des modes de réalisation du dispositif selon l'invention peut être réalisé en toute matière déjà utilisée dans les sondes respiratoires, par exemple en un chlorure de polyvinyle, avec un éventuel revêtement de silicone ou en acier permettant les injections à pression élevée.

Bien entendu, les dimensions du dispositif selon l'invention peuvent être très variables, essentiellement en fonction de la voie de mise en place du tube, et de la taille du patient, qui peut être un adulte, un enfant, un nourrisson ou un prématuré.

## Revendications

1. Dispositif d'assistance respiratoire comportant un tube (4) qui forme un canal principal (5) et qui est destiné à être relié par son extrémité distale (3) à une voie respiratoire d'un patient pour que ledit canal principal (5) relie à l'extérieur le système respiratoire dudit patient, ledit dispositif comportant de plus au moins un canal auxiliaire (8,8.1) ménagé dans la paroi dudit tube (4) et permettant l'injection d'un jet gazeux destiné à la ventilation dudit patient et débouchant dans ledit canal principal (5) au voisinage de l'extrémité distale (7) de ce dernier,
caractérisé en ce que au moins l'extrémité distale dudit canal auxiliaire (8,8.1) débouchant dans le canal principal (5) est parallèle à celui-ci, en ce que ledit orifice distal (17) dudit canal auxiliaire (8,8.1) est formé dans une première face (14a) s'écartant de l'axe dudit canal principal (5), et en ce que, en regard de l'orifice distal (17) dudit canal auxiliaire (8,8.1) sont prévus des moyens (14b) pour la déflexion dudit jet de gaz respirable de ventilation vers l'intérieur dudit canal principal (5), lesdits moyens de déflexion étant formés par une seconde face (14b), inclinée, disposée en regard de ladite première face et convergente en direction de l'orifice distal (7) dudit canal principal (5).

2. Dispositif selon la revendication 1, dans lequel on prévoit plusieurs canaux auxiliaires (8,8.1) ménagés dans la paroi dudit tube (4),
caractérisé en ce que les orifices distaux (17) desdits canaux auxiliaires (8,8.1) sont formés dans une première face annulaire (14a) évasant ledit canal principal (5) et en ce que lesdits moyens de déflexion sont formés par une seconde face annulaire (14b), disposée en regard de ladite première face et convergente en direction de l'orifice distal (7) dudit canal principal.

3. Dispositif selon l'une quelconque des revendications 1 ou 2,
caractérisé en ce que ladite seconde face (14b) est prolongée vers ledit orifice distal (7) du canal principal (5) par une paroi évasant légèrement ledit canal principal (5).

4. Dispositif selon l'une quelconque des revendications 1 à 3,
caractérisé en ce que lesdits moyens de déflexion sont formés sur un embout rapporté à l'extrémité distale dudit tube (4).

5. Dispositif selon la revendication 1,
caractérisé en ce que les moyens de déflexion consistent en un ensemble discontinu d'évidements de forme générale conique, ménagés dans la paroi interne 15, et au fond de chacun desquels débouche l'extrémité distale d'un canal auxiliaire 8.

6. Dispositif selon la revendication 1,
caractérisé en ce qu'il comporte une pluralité de canaux auxiliaires (8,8.1), dont au moins certains sont alimentés en commun en gaz respirable sous pression.

7. Dispositif selon la revendication 6,
caractérisé en ce que l'alimentation en commun desdits canaux se fait par l'intermédiaire d'une bague de distribution (9), coaxiale audit tube (4).

8. Dispositif selon l'une des revendications 5 ou 6,
caractérisé en ce que lesdits canaux auxiliaires (8,8.1) non alimentés en commun, servent à l'introduction de produits gazeux additionnels, tels que des produits médicamenteux ou des gaz humides.

9. Dispositif selon la revendication 1,
caractérisé en ce qu'il comporte au moins un canal auxiliaire supplémentaire (8.2), indépendant dudit canal auxiliaire (8) et relié à une source de gaz sous pression, ledit canal auxiliaire supplémentaire débouchant dans ledit canal principal (5) au voisinage de l'extrémité proximale (7.2) de ce dernier, en ce qu'au moins l'extrémité proximale dudit canal auxiliaire supplémentaire (8.2) débouchant dans le canal principal (5) est parallèle à celui-ci et en ce que, en regard de l'orifice proximal dudit canal auxiliaire supplémentaire (8.2), sont prévus des moyens pour la déflexion du jet de gaz traversant ce dernier en direction de l'axe dudit canal principal (5).

10. Dispositif selon la revendication 9,
caractérisé en ce que la partie proximale (1.2) du dispositif (22) est semblable sinon identique, à la partie distale (1.1) dudit dispositif, au moins en ce qui concerne la disposition desdits canaux auxiliaires et lesdits moyens de déflexion.

11. Dispositif selon la revendication 1,
caractérisé en ce qu'il constitue l'embout d'entrée et de sortie d'air d'un masque d'assistance respiratoire destiné à être appliqué sur le visage d'un patient.

12. Masque d'assistance respiratoire,
caractérisé en ce qu'il est muni d'un dispositif selon la revendication 11.

## Claims

1. Respiratory assistance device comprising a tube (4) which forms a main channel (5) and which is designed to be connected by its distal end (3) to a respiratory tract of a patient so that the said main channel (5) connects to the outside the respiratory system of the said patient, the said device additionally comprising at least one auxiliary channel (8, 8.1) made in the wall of the said tube (4) and permitting the injection of a gaseous jet intended for the ventilation of the said patient and opening into the said main channel (5) in the vicinity of the distal end (7) of the latter, characterised in that at least the distal end of the said auxiliary channel (8, 8.1) opening into the main channel (5) is parallel to the latter, in that the said distal aperture (17) of the said auxiliary channel (8, 8.1) is formed in a first surface (14a) diverging from the axis of the said main channel (5), and in that, facing the distal aperture (17) of the said auxiliary channel (8, 8.1) are provided means (14b) for deflecting the said jet of breathable gas for ventilation towards the interior of the said main channel (5), the said deflection means being formed by a second surface (14b), sloping, disposed facing the said first surface and converging in the direction of the distal aperture (7) of the said main channel (5).

2. Device according to Claim 1, in which there are provided several auxiliary channels (8, 8.1) made in the wall of the said tube (4) characterised in that the distal apertures (17) of the said auxiliary channels (8, 8.1) are formed in a first annular surface (14a) widening the said main channel (5) and in that the said deflection means are formed by a second annular surface (14b) disposed facing the said first surface and converging in the direction of the distal aperture (7) of the said main channel.

3. Device according to either of Claims 1 or 2, characterised in that the said second surface (14b) is extended towards the said distal aperture (7) of the main channel (5) by a wall widening slightly the said main channel (5).

4. Device according to any one of Claims 1 to 3, characterised in that the said deflection means are formed on an end piece added to the distal end of the said tube (4).

5. Device according to Claim 1, characterised in that the deflection means consist of a discontinuous set of hollowed-out spaces of conical general shape, made in the internal wall 15, and at the bottom of each of which opens the distal end of an auxiliary channel 8.

6. Device according to Claim 1, characterised in that it comprises a plurality of auxiliary channels (8, 8.1), at least some of which are jointly supplied with breathable gas under pressure.

7. Device according to Claim 6, characterised in that the joint supply of the said channels is provided through the intermediary of a distribution ring (9), coaxial to the said tube (4).

8. Device according to either of Claims 5 or 6, characterised in that the said auxiliary channels (8, 8.1) not jointly supplied, serve for the admission of additional gaseous products, such as medicamentary products or moist gases.

9. Device according to Claim 1, characterised in that it comprises at least one additional auxiliary channel (8.2), independent of the said auxiliary channel (8) and connected to a source of gas under pressure, the said additional auxiliary channel opening into the said main channel (5) in the vicinity of the proximal end (7.2) of the latter, in that at least the proximal end of the said additional auxiliary channel (8.2) opening into the main channel (5) is parallel to the latter and in that, facing the proximal aperture of the said additional auxiliary channel (8.2), means are provided for the deflection of the jet of gas passing through the latter in the direction of the axis of the said main channel (5).

10. Device according to Claim 9, characterised in that the proximal part (1.2) of the device (22) is similar, if not identical, to the distal part (1.1) of the said device, at least as far as the disposition of the said auxiliary channels and the said deflection means is concerned.

11. Device according to Claim 1, characterised in that it constitutes the air inlet and outlet end piece of a respiratory assistance mask designed to be applied onto the face of a patient.

12. Respiratory assistance mask, characterised in that it is provided with a device according to Claim 11.

## Patentansprüche

1. Vorrichtung zur Unterstützung der Atmung mit einem Rohr (4), das einen Hauptkanal (5) bildet und das dazu bestimmt ist, mit seinem distalen Ende (3) mit einem Atemweg eines Patienten verbunden zu werden, wofür der Hauptkanal (5) das Atmungssystem des Patienten nach draußen verbindet, wobei die Vorrichtung außerdem wenigstens einen Hilfskanal (8,8.1) umfaßt der in der Seitenwand des Rohres (4) angebracht ist und die Injektion eines zur Beatmung eines Patienten bestimmten Gasstroms erlaubt und der in den Hauptkanal (5) in der Nähe dessen distalen Endes (7) mündet,
dadurch gekennzeichnet, daß wenigstens das distale Ende des Hilfskanals (8,8.1), das in den Hauptkanal (5) mündet, zu diesem parallel steht, und dadurch, daß die distale Öffnung (17) des Hilfskanals (8,8.1) in einer ersten Fläche (14a) gebildet wird, die von der Achse des Hauptkanals (5) abweicht, und dadurch, daß gegenüber der distalen Öffnung (17) des Hilfskanals (8,8.1) Mittel (14b) zur Ablenkung des Beatmungsgasstroms zur Beatmung im Inneren des Hauptkanals (5), vorgesehen sind, wobei die Ablenkmittel durch eine zweite Fläche (14b) gebildet werden, die geneigt, gegenüber der ersten Fläche angeordnet und in Richtung der distalen Öffnung (7) des Hauptkanals (5) konvergent ist.

2. Vorrichtung nach Anspruch 1, in der mehrere Hilfskanäle (8,8.1) vorgesehen sind, die in der Seitenwand des Rohres (4) angebracht sind,
dadurch gekennzeichnet, daß die distalen Öffnungen (17) der Hilfskanäle (8,8.1) in eine erste ringförmige Fläche (14a) geformt sind, die den Hauptkanal (5) ausweitet, und dadurch, daß die Ablenkmittel durch eine zweite ringförmige Fläche (14b) gebildet sind, die gegenüber der ersten Fläche angebracht ist und in Richtung auf die distale Öffnung (7) des Hauptkanals konvergiert.

3. Vorrichtung nach einem der Ansprüche 1 oder 2
dadurch gekennzeichnet, daß die zweite Fläche (14b) zur distalen Öffnung (7) des Hauptkanals (5) hin durch eine Wand verlängert ist, die den Hauptkanal (5) leicht ausweitet.

4. Vorrichtung nach einem der Ansprüche 1 bis 3
dadurch gekennzeichnet, daß die Ablenkmittel durch einen Ansatz gebildet werden, der auf das distale Ende des Rohres (4) aufgesetzt ist.

5. Vorrichtung nach Anspruch 1
dadurch gekennzeichnet, daß die Ablenkmittel aus einer diskontinuierlich angeordneten Menge von konisch geformten Aussparungen besteht, die in der inneren Seitenwand (15) angebracht sind und am Boden jeder Aussparung die distale Öffnung eines Hilfskanals (8) ausmündet.

6. Vorrichtung nach Anspruch 1
dadurch gekennzeichnet, daß sie eine Mehrzahl von Hilfskanälen (8,8.1) umfaßt, von denen wenigstens einige Bestimmte gemeinsam mit einem unter Druck stehenden Beatmungsgas versorgt sind.

7. Vorrichtung nach Anspruch 6
dadurch gekennzeichnet, daß die gemeinsame Versorgung der Kanäle mittels eines koaxial zum Rohr (4) stehenden Verteilerringes (9) geschieht.

8. Vorrichtung nach einem der Ansprüche 5 oder 6
dadurch gekennzeichnet, daß die nicht gemeinsam versorgten Hilfskanäle (8,8.1) zur Einführung von zusätzlichen gasförmigen Produkten, wie Heilmittelprodukte oder Befeuchtungsgase, dienen.

9. Vorrichtung nach Anspruch 1
dadurch gekennzeichnet, daß sie wenigstens einen Hilfskanal (8.2) umfaßt, der unabhängig vom Hilfskanal (8) ist und mit einer unter Druck stehenden Gasversorgung verbunden ist, wobei der zusätzliche Hilfskanal in den Hauptkanal (5) in der Nähe von dessen proximalen Ende (7.2) mündet, und dadurch, daß wenigstens das proximale Ende des zusätzlichen Hilfskanals (8.2), das in den Hauptkanal (5) mündet, parallel zu diesem steht, und dadurch, daß gegenüber der proximalen Öffnung des zusätzlichen Hilfskanals (8.2) Mittel zur Ablenkung des Gasstroms, der diesen in Richtung der Achse des Hauptkanals (5) durchströmt, vorgesehen sind.

10. Vorrichtung nach Anspruch 9
dadurch gekennzeichnet, daß der proximale Teil (1.2) der Vorrichtung (22) mit dem distalen Teil (1.1) der Vorrichtung wenigstens im Hinblick auf das, was die Anordnung der Hilfskanäle und der Ablenkmittel betrifft, ähnlich, wenn nicht gar identisch ist.

11. Vorrichtung nach Anspruch 1
dadurch gekennzeichnet, daß sie den Luftein- und Austrittsstutzen einer Maske zur Unterstützung der Beatmung darstellt, die dazu bestimmt ist, auf dem Gesicht eines Patienten angelegt zu werden.

12. Maske zur Unterstützung der Atmung
dadurch gekennzeichnet, daß sie mit einer Vorrichtung nach Anspruch 11 versehen ist.
